(19)

Europäisches
Patentamt
European
Patent Office
Office européen
des brevets

(11)     **EP 1 199 984 B1**

(12)                        **EUROPEAN PATENT SPECIFICATION**

(45) Date of publication and mention
of the grant of the patent:
**07.11.2007   Bulletin 2007/45**

(51) Int Cl.:
***A61B 6/00*** (2006.01)

(21) Application number: **01922702.4**

(22) Date of filing: **26.03.2001**

(86) International application number:
**PCT/US2001/009690**

(87) International publication number:
**WO 2001/080739 (01.11.2001 Gazette 2001/44)**

(54) **AUTOMATING USER CUSTOMIZATION OF A MEDICAL DEVICE**

AUTOMATISCHE BENUTZERPERSONALISIERUNG EINES MEDIZINISCHEN GERÄTS

AUTOMATISER LA PERSONNALISATION SELON L'UTILISATEUR D'UN DISPOSITIF MEDICAL

(84) Designated Contracting States:
**DE NL**

(30) Priority: **26.04.2000  US 558660**

(43) Date of publication of application:
**02.05.2002   Bulletin 2002/18**

(73) Proprietor: **GE Medical Systems Global
Technology Company LLC
Waukesha,
Wisconsin 53188 (US)**

(72) Inventors:
• **CHILDRESS, Frank, Willard
Brookfield, WI 53018 (US)**
• **UNGER, Christopher, David
Delafield, WI 53018 (US)**

(74) Representative: **Goode, Ian Roy
London Patent Operation
General Electric International, Inc.
15 John Adam Street
London WC2N 6LU (GB)**

(56) References cited:
• **PATENT ABSTRACTS OF JAPAN vol. 013, no. 149
(P-855), 12 April 1989 (1989-04-12) -& JP 63 310078
A (FUJITSU LTD), 19 December 1988 (1988-12-19)**
• **PATENT ABSTRACTS OF JAPAN vol. 014, no. 131
(C-0700), 13 March 1990 (1990-03-13) -& JP 02
004350 A (FUJITSU LTD), 9 January 1990
(1990-01-09)**
• **PATENT ABSTRACTS OF JAPAN vol. 1998, no.
14, 31 December 1998 (1998-12-31) -& JP 10
234730 A (SHIMADZU CORP), 8 September 1998
(1998-09-08)**

**Description**

BACKGROUND OF THE INVENTION

The present invention generally relates to adjusting the settings on a medical device. More particularly, the present invention relates to an automated system that configures the medical device based on the user's preset preferences.

Medical devices such as X-ray systems or monitoring systems have several user selectable adjustments. These adjustments include table height, spatial or temporal filtering, screen layouts, and the like. Since it is common for such machines to be used in a hospital, where numerous doctors have access to and use the machine, each doctor must manually configure these settings to the doctor's own preferences before each use.

Conventional automated systems simply adjust the machine settings based on the doctor's default preferences. Conventional automated systems contain databases that store the doctor's preferences. Many types of prior art systems would not update the doctor's preferences unless the doctor went through the time consuming task of entering the database and manually changing the setting. Oftentimes the system required a technician to manually adjust the setting on the machine. Thus, prior art systems did not allow for easy updating of the default system settings if a doctor's preference changed over time.

In other conventional systems the default setting reset after every use. Once the system recognized the user, the system adjusted the system settings to the value last used by the doctor. However, these prior art systems did not take into account the need for a doctor to adjust the system in accordance with a particular patient's needs. Some machine settings are based on traits such as the patient's weight, height or build. Doctors often have to adjust a machine in order to meet the needs of each individual patient. Since it is unlikely that two consecutive patients will have exactly the same traits, the doctor will still have to manually adjust the settings to meet the needs of the next patient. Conventional systems do not take into account the reason why the doctor changed the system setting, nor consider the extent of the variation in the system settings.

JP 63 31 00 78 describes a medical diagnostic device set by reading an IC card of a patient and ID of a doctor.

JP 02 00 43 50 describes an ultrasound diagnosing device in which a newest parameter setting is kept and used when a mode is again selected.

Patent Abstract of Japan Vol. 1998, no 14, 31 Dec 1998 and JP 10 23 47 30 describe an ultrasound diagnosing device in which past data of a patient can be brought to a doctor.

Thus, there has been a long felt need in the medical field for an automated system that configures a medical device, monitors a doctor's use and updates the default system settings in accordance with consistent changes made by the doctor. The preferred embodiments of the present invention address these needs and other concerns.

BRIEF SUMMARY OF THE INVENTION

A first aspect of the present invention is described in appended claim 1.

A second aspect of the present invention is described in appended claim 9.

The preferred embodiments of the present invention provide an automated system and method of use therefor that configures a medical device to a doctor's presences without having to manually adjust the settings before each use. The system utilizes a user recognition module to identify a particular user. Once the system recognizes the user, the system automatically accesses a database containing the preset default preferences for that individual user. The preset default preferences are then downloaded to a controller that adjusts the medical device settings in accordance with these defaults.

The automated system also updates the preset default preferences to comply with the doctor current preferences. The controller includes a computer that monitors each time the doctor uses a particular function and makes a record of each instance in which the doctor changes a default setting. When such a change occurs, the computer first determines whether the doctor is overriding a preset default setting. Doctors are often faced with situations in which they must adapt a system to meet a patient's particular needs, such as when doctors are dealing with an unusually heavy patient or a very young, small patient. In such a situation, the doctor must adjust the medical device's settings to effectively treat or diagnose the patient. However, it is unlikely that the doctor will want this single change to affect his or her preset default settings. The system receives patient information, such as the patient's weight, which allows it to recognize when the doctor is treating a patient with unusual characteristics. If the system determines that the patient has unusual characteristics, the system will not consider any changes to the settings as an override, and therefore, not adjust the preset default settings.

However, if the system determines that the doctor is not treating a patient with irregular needs, the system will identify the change as an override of the default settings and update the preset default settings accordingly. In updating the default settings, the system identifies which parameters are being adjusted. The system supports at least two types of settings, namely 1) those that depend on the doctor's preferences and 2) those that depend on the characteristics of a particular patient. Parameters that relate primarily to a doctor's particular preferences include items such as table height (which corresponds to the height of the doctor) or table side control configuration (which is determined by whether the doctor is right-handed or left-handed) (hereinafter user-specific traits). Since the traits rarely change, the default setting parameters associated solely or primarily with user-specific traits will remain fairly constant. Thus, if a doctor overrides one of the user-specific traits, it is likely that the override reflects a change in the doctor's preference, and therefore, the system will reset the default settings to comply with the new setting.

However, a slightly more complex process arises when a parameter depends on the patient's characteristics, such as the patient's height, weight or build. Unlike the doctor's preferences, device parameters associated with patient characteristics may be adjusted frequently. A change in one patient dependant parameter usually reflects a doctor's attempt to adapt the device to the needs of a particular patient rather that the doctor's own preferences. Thus, rather than resetting the user's preset default preferences, the system calculates a new default parameter setting using pre-programmed algorithms. The pre-programmed algorithms limit the effect of a single override based on the characteristics of the parameter, such as whether the parameter is a continuous or discrete variable.

Continuous variables, such as spatial blurring or sharpening, may be set at one of many different values within a given range. If the doctor overrides a continuous variable, the system will use a filtered algorithm to update the variable, such as an algorithm that compares the average value for a specified number of uses (i.e. the average setting over the last 10 patients) and updates the default setting for the variable only if the variation between the average and the default setting is statistically significant. In this manner, the system monitors long-term trends in presences without allowing short-term changes made by the doctor to skew the default settings.

Discrete variables, on the other hand, may only be set to a limited number of values, such as a switch that may either be turned on or off. Since the values for discrete variables are limited, it is nearly impossible to calculate an average default value (a switch cannot be 65% on). Thus, to prevent single changes from greatly altering the default setting, the system updates the default settings only if a particular value for a discrete variable is used a preset number of times, such as 3 out of the last 4. Thus, regardless of the parameter characteristics or the variable type, the system adjusts the setting default value to accurately reflect the doctor's current preferences.

These and other features of the present invention are discussed in the following detailed description of the preferred embodiments of the present invention. It shall be understood that other features and advantages will become apparent to those skilled in the art upon review of the following detailed description, drawings, and claims.

BRIEF DESCRIPTION OF THE DRAWINGS

Figure 1 illustrates an apparatus for automating user-customization of a medical device in accordance with a preferred embodiment of the present invention.

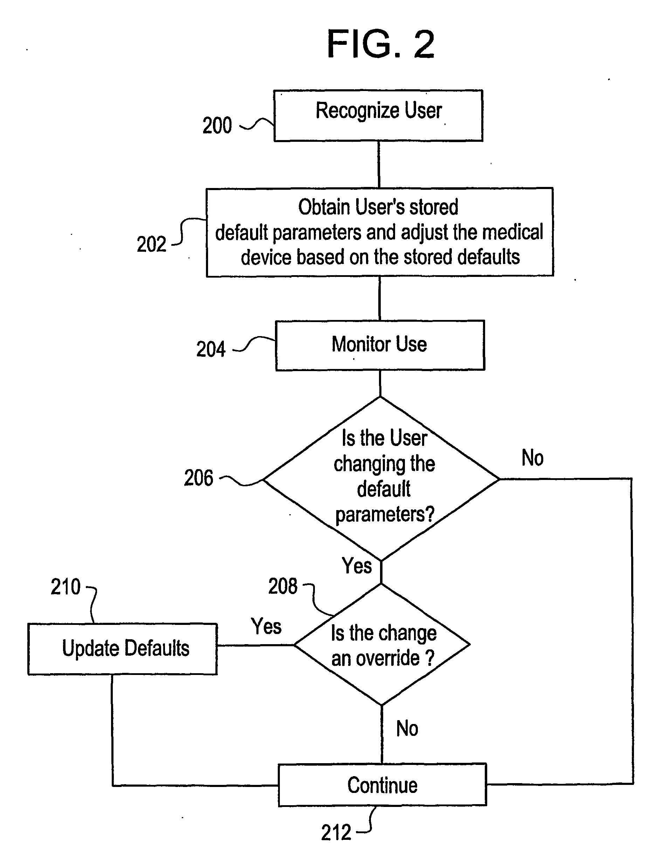

Figure 2 illustrates a flowchart outlining the steps carried out by the system in accordance with a preferred embodiment of the present invention in order to set the parameters and variables of the medical device.

DETAILED DESCRIPTION OF THE INVENTION

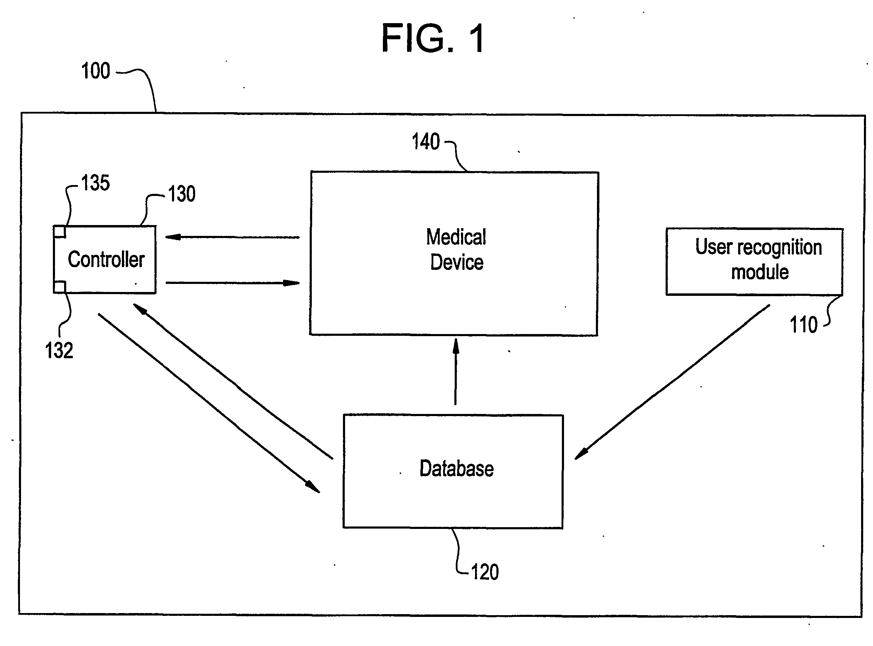

Figure 1 illustrates a schematic block diagram of an automated system in accordance with a preferred embodiment of the present invention for updating the parameter settings of a medical device. The system 100 includes a user recognition module 110, a database 120 and a controller 130 for monitoring, calculating and controlling the parameter settings for a medical device 140. The system 100 is compatible with any medical device that contains adjustable parameters, such as X-ray, ultrasound, MRI, CT, nuclear medicine, medical imaging equipment and the like.

At step 200, the user (usually a doctor) wishing to use the medical device simply identifies himself or herself to the system via the user recognition module 110. The user recognition module 110 may implement several different methods that are well known in the art to identify the user, including through a key or tag worn by the doctor, voice recognition, retinal pattern recognition or use of a user login or password. The type of user recognition performed by the module 110 depends on the system technology available and the normal system operation. Since doctors often wear identification badges, one preferred embodiment may implement an identification mechanism within the user's badge.

Once the user recognition module 110 recognizes the user, the system 100 accesses the database 120 that stores a plurality of user default preferences for one or more parameters of the corresponding medical device (step 202). By way of example, the database 120 may store one or more user default preferences for each doctor that uses a particular medical device 140. Each user default preference may comprise values for a set of parameters, such as table height, table side control configuration, spatial blurring, image contrast and sharpness parameters and the like. The user default preferences for a particular user may include a set of values discrete and/or continuous parameters, representing a set of default settings.

The doctor may either enter the data prior to the first use, or the settings entered during the first use may automatically become the default settings. Also, the format of the database may vary. For example, the database 120 may be quite simple, with the user ID as the database key or index and the parameters listed as fields under that index. Alternatively, the database 120 may be organized with a standard ".ini" format, or (name, value) format. Furthermore, the database can be divided into several subdivisions per user. In this manner, doctors may enter separate user default preferences for a single machine, such as differing user default preferences when a doctor is X-raying a hand as opposed to a chest. The stored user default preferences are then downloaded to the controller 130 which in turn automatically adjusts the settings for the parameters of the medical device 140 in accordance with a selected set of user default preferences.

An alternate embodiment may include a database 120 that is remotely located from the medical device 140. In order to download the settings, the medical device 140 communicates with the database 120, such as via the internet, an intranet, local area network, wide area network or the like. Doctors who practice at multiple hospitals or offices may easily access user default preferences regardless of his or her location. Furthermore, the default settings can be entered and/or changed immediately without requiring a doctor or third party to be present at the medical device 140.

Also, the use of a database 120 at a remote location may allow a single database 120 to store particular user's default preferences that are common to numerous medical devices. Many medical devices have common settings such as table height and table side control configuration. In this embodiment the database 120 is in communication with numerous machines, and thus, the system 100 may update these common settings in accordance with the user's most recent parameter settings regardless of what medical device is being used.

Once the medical device 140 is configured, the doctor may begin to use it. The controller 130 includes a computer 132 that monitors use of the medical device 140 in order to adjust and update the doctor's user default preferences in accordance with the doctor's most recent parameter entries (step 204). During monitoring, the computer 132 recognizes each adjustment by the doctor to parameter values or settings for the medical device 140 (step 206). Once the computer 132 recognizes a change, the computer 132 determines if the user is "overriding" the user default preferences (step 208). The computer 132 may use any of several different ways to identify overrides, including a simple preference capture routine that defines an override as any occasion in which the user adjusts any parameter. For example, a simple preference capture routine may direct the computer 132 to update the user default preferences each time that the user changes a parameter.

Alternatively, the computer 132 may utilize a more complex preference capture routine that analyzes and recognizes multiple reasons why the doctor may be adjusting the parameter setting. The "smart" preference capture routine prevents the computer 132 from updating the preferences too often. Once the computer 132 recognizes that the user is changing the parameter setting, the computer 132 decides whether to adjust the user default preference in accordance with the user's action based upon the parameter setting that the doctor is adjusting and based upon the environment and patient characteristics. Since the preferred embodiment of the present invention may be used with various medical devices with adjustable parameter settings, each specific parameter is not listed herein. However, in several medical devices, the parameters may be classified into one of two categories by the system 100, namely parameters that depend on the doctor's personal preferences and parameters that depend on the particular patient's characteristics.

Certain parameter settings relate directly to the doctor's likes or dislikes. Settings such as table height or table side control configuration depend on the doctor's height and on whether the doctor is left handed or right handed. Since these traits typically do not change from examination to examination, if a doctor changes one of these settings the computer 132 assumes that the doctor is overriding his or her default. Accordingly, processing passes to step 210, at which the computer 132 updates the user default preferences in the database 120.

On the other hand, settings such as spatial or temporal filtering strength depend more specifically on the patient's traits, such as the patient's build or weight. A doctor may have to adjust the amount of filtering performed by the medical device 140 to adapt to a specific situation (such as an unusually heavy patient, or a very young, small patient). It is unlikely that the next patient will be of the same relative height, weight and build. Thus, the doctor typically will not need the medical device 140 to return to the settings needed for the previous patient, especially if the previous patient had unusual characteristics. In other words, if the doctor is treating a patient having one or more unusual characteristics, the computer 132 should not consider any change made by the doctor to any parameter setting as an override at step 208. Hence, processing passes to step 212.

The computer 132 is provided with patient characteristic information at or before step 208. Based on the patient characteristic information, the computer 132 determines if a parameter change constitutes an override. The computer 132 may obtain the patient characteristic information in several manners. For example, the computer 132 may access patient characteristic information from either a hospital or remote database. Since it is common practice for a doctor to receive information such as weight before treating a patient, this information may be easily obtained. If it was not hospital policy to obtain patient characteristic information prior to treatment, the medical device 140 may also include one or more sensors in order to obtain the patient characteristic information, such as weight. If the computer 132 determined that the patient's weight did not fall within a preset range, the computer would not update the user default preferences based on the changes made during the particular treatment.

Alternatively, the controller 130 may provide the doctor with an override switch 135. The switch 135 controls the inputs to the medical device 140 and may specifically control one or more parameter settings. If the doctor desired to adjust a parameter setting but did not want that single change to affect his or her user default preferences, the doctor could simply engage the override switch, notifying the computer 132 not to update the user default preferences based on the present change.

Optionally, the computer 132 may monitor the time at which a particular parameter adjustment took place. In most situations, a doctor adjusts the medical device 140 to his or her liking prior to using the medical device. Thus, the computer 132 may assume that any adjustment made after an examination has commenced is based on the patient's needs rather that a change to the doctor's preferences, and thus will not be considered to override the user default preferences.

When the computer 132 determines that the doctor is overriding a parameter setting, the computer 132 updates the user default preferences accordingly (step 210). Numerous database update routines are well-known, and thus, the preferred embodiments are not limited to any particular methods of recalculating or updating the user default preferences. In the preferred embodiment, the computer 132 identifies whether a particular parameter is a continuous or discrete variable. Continuous variables may be set at one of many different values. Examples include table height or spatial filtering, that may be set at any value within a given range. Since these variables may be adjusted to several different values, a filtered algorithm may be used to determine consistent patterns in a doctor's use. The following are examples of such algorithms.

According to a simple algorithm, the computer 132 continuously filters the user selections, such as a simple alpha filter:

$$\text{Default}_{NEW} = \alpha * \text{Default}_{OLD} + (1-\alpha) * \text{User\_Selection}$$

where $\text{Default}_{NEW}$ is the new default setting, $\text{Default}_{OLD}$ is the previous default setting, User-Selection is the user's input value and $0 \# \alpha < 1$. In the above equation, alpha ($\alpha$) may equal zero (in which case the computer would reset the default setting after every use), or any value up to one (in which case the computer would never update the default setting). The higher the value of alpha, the less effect the new value will have on the setting for the particular user default preference.

In using the above equation, each parameter may be assigned its own alpha value by the doctor depending on how drastic a change the user desires after every use. For example, parameters that depend primarily on the doctor's preferences would have a very low alpha value, even zero. As mentioned above, parameters such as table height or table side configuration rarely change, if at all. Thus, a change by the doctor likely reflects a change in personal preference. For example, if a doctor changed the table side control configuration (say because the doctor developed arthritis in his dominant hand), the system 100 may assume that the doctor would like this same configuration in all future exams. Thus, if the computer 132 recognizes that the doctor is changing one of these parameters, the computer 132 should reset the user default preferences to comply with the doctor's new preference.

Alternatively, other parameters depend more on the patient's characteristics, and therefore, may change more frequently. In updating patient dependent parameters, the user will use a much higher value of alpha to prevent the computer 132 from updating the default settings after every use. If the user determines that the assigned alpha value results in either too much or too little deviation in a default setting, the user can readily change the alpha value for the given setting accordingly.

A slightly more complex alternative for updating continuous variable would monitor the typical magnitude of the variation of the overrides:

$$\text{NewAvg}_{NEW} = \alpha_1 * \text{NewAvg}_{OLD} + (1-\alpha_1) * \text{User\_Selection};$$

$$\text{Var'n}_{NEW} = \alpha_2 * \text{Var'n}_{OLD} + (1-\alpha_2) * (\text{User\_Selection} - \text{NewAvg});$$

$$\text{If (NewAvg} - \text{Default)/Var'n} > 3.0, \text{ then Default} = \text{NewAvg}$$

where $NewAvg_{NEW}$ is the average including the present override value, $NewAvg_{OLD}$ is the previous average value (i.e. not including the present override value), User_Selection is the present override value, $Var'n_{NEW}$ and $Var'n_{OLD}$ are the differences between the default and the new and old averages respectively, $0 < \alpha_1 < 1$ and $0 < \alpha_2 < 1$.

According to this alternative algorithm, the computer 132 updates the user default preferences only if the difference between the default and the current operating average is statistically significant (for example, designated in the above equation as 3.0). The user may adjust the level of "statistical significance" in order to allow the computer 132 to update the default more or less frequently.

Discrete variables, on the other hand, may only be set to a very limited number of values, such as a switch being on or off, on a setting for a right-handed or left-handed person. Since the number of values is limited, it is much easier to determine whether or not a doctor is overriding the value. However, it is much more difficult to determine whether that change is significant enough to warrant adjusting the default value. The computer 132 cannot simply calculate an average value for the setting. A simple algorithm could be used based on the number of times a particular value is used, such as 3 out of 5 times. A different numerical requirement could be used based on the number of settings available to the user. Again, although many other algorithms are well-known in the art, the exact algorithm used in updating these variables may vary, and thus additional algorithms will not be described herein.

**Claims**

1.  An automated system (100) for controlling parameter settings of a medical device (140) comprising:

    a medical device (140) including adjustable settings;
    a user recognition module (110) operable to identify a user;
    a database (120) for storing at least one parameters setting associated with a particular user's preferences for a configuration of said medical device (140);
    a controller (130) for automatically adjusting said medical device (140) in accordance with a particular user's preference settings based on said at least one parameter setting; and
    a computer (132) operable to recognize each time the user overrides the user's preference settings and updates said at least one parameter setting based upon a value of a parameter override, **characterized in that**:

    the computer is operable to recognize the user overrides by determining if a parameter change constitutes an override based upon patient characteristic information.

2.  The automated system (100) of claim 1, further comprising an override switch (135) operable to instruct said controller (130) whether to adjust said at least one parameter setting when the user changes a parameter setting.

3.  The automated system (100) of claim 1 wherein said database (120) is remotely located from the medical device (140).

4.  The automated system (100) of claim 3 wherein said database (120) is operable to communicate with the medical device (140) via the internet.

5.  The automated system (100) of claim 3 wherein said database (120) is operable to store a single user preference setting for settings that are common to multiple medical devices.

6.  The automated system (100) of claim 1 wherein said parameter settings are divided into multiple categories, at least one category of which contains parameters dissociated with user-specific traits and another category of which contains parameters associated with patient-dependent characteristics, and wherein said computer (132) is operable to recalculate said user's preference setting differently for each category.

7.  The automated system (100) or claim 1 wherein setting parameters are either continuous or variable, and wherein said computer (132) is operable to recalculate said user's preference setting differently for each type of parameter.

8.  The automated system (100) of claim 7 wherein continuous parameters are updated using an alpha filter.

9. A method for automatically controlling at least one parameter setting of a medical device (140) associated with an individual user preference for a medical device (140), comprising:

identifying a particular user;
obtaining at least one default parameter setting associated with preferences for the identified user;
adjusting the settings of the medical device (140) to conform to the user preferences:

monitoring a present state of parameter settings for the medical device (140);
recognizing when the parameter settings are changed for the said medical device (140);
determining whether a parameter change constitutes an override of a default parameter setting; and
when a change constitutes an override, updating said at least one parameter setting in accordance with the change, **characterized in that**:

determining whether a parameter change constitutes an override of a default parameter setting is based upon patient characteristic information.

10. The method of claim 9 wherein said the parameter settings are divided into multiple categories, at least one category of which contains parameters associated with user-specific traits and another category of which contains parameters associated with patient-dependent characteristics, and a computer (132) recalculates said user's preference setting differently for each category.

11. The method of claim 9 wherein setting parameters are either continuous or variable, and a computer (132) recalculates said user's preference setting differently for each type of parameter.

12. The method of claim 11 wherein continuous parameters are updated using an alpha filter.

**Patentansprüche**

1. Automatisches System (100) zum Steuern von Parametereinstellungen eines medizinischen Gerätes (140), aufweisend:

ein medizinisches Gerät (140) mit anpassbaren Einstellungen;
ein Benutzererkennungsmodul (110), das zum Identifizieren eines Benutzers betrieben werden kann;
eine Datenbank (120) zum Speichern wenigstens einer Parametereinstellung in Verbindung mit Präferenzen eines speziellen Benutzers für eine Konfiguration des medizinischen Gerätes (140);
eine Steuerung (130) zum automatischen Anpassen des medizinischen Gerätes (140) gemäß Präferenzeinstellungen eines speziellen Benutzers auf der Basis der wenigstens einen Parametereinstellung; und
einen Computer (132), der so betrieben werden kann, dass er jedes mal erkennt, wenn der Benutzer die Benutzerpräferenzeinstellungen überschreibt, und die wenigstens eine Parametereinstellung auf der Basis eines Wertes einer Parameterüberschreibung aktualisiert,
**dadurch gekennzeichnet, dass**:

der Computer so betrieben werden kann, dass er die Benutzerüberschreibungen erkennt, indem er ermittelt, ob eine Parameteränderung auf der Basis von patientencharakteristischer Information eine Überschreibung darstellt.

2. Automatisches System (100) nach Anspruch 1, welches ferner einen Überschreibungsschalter (135) aufweist, der betätigt werden kann, um die Steuerung (130) anzuweisen, ob sie die wenigstens eine Parametereinstellung anpassen soll, wenn der Benutzer eine Parametereinstellung ändert.

3. Automatisches System (100) nach Anspruch 1, wobei sich die Datenbank (120) an einer von dem medizinischen Gerät (140) entfernten Stelle befindet.

4. Automatisches System (100) nach Anspruch 3, wobei die Datenbank (120) so betrieben werden kann, dass sie mit dem medizinischen Gerät (140) über das Internet kommuniziert.

5. Automatisches System (100) nach Anspruch 1, wobei die Datenbank (120) so betrieben werden kann, dass sie die Präferenzeinstellung eines einzelnen Benutzers für Einstellungen speichert, die für mehrere medizinische Geräte üblich sind.

6.  Automatisches System (100) nach Anspruch 1, wobei Parametereinstellungen in mehrere Kategorien unterteilt sind, wovon wenigstens eine Kategorie Parameter in Verbindung mit benutzerspezifischen Eigenheiten enthält und eine weitere Kategorie davon Parameter in Verbindung mit patientenabhängigen Eigenschaften enthält, und wobei der Computer (132) so betrieben werden kann, dass er die Benutzerpräferenzeinstellung für jede Kategorie unterschiedlich neu berechnen kann.

7.  Automatisches System (100) nach Anspruch 1, wobei die Einstellparameter entweder gleich bleibend oder variabel sind, und wobei der Computer (132) so betrieben werden kann, dass er die Benutzerpräferenzeinstellung für jeden Parametertyp unterschiedlich neu berechnet.

8.  Automatisches System (100) nach Anspruch 7, wobei gleich bleibende Parameter unter Verwendung eines Alpha-Filters aktualisiert werden.

9.  Verfahren zum automatischen Steuern wenigstens einer Parametereinstellung eines medizinischen Gerätes (140) in Verbindung mit einer individuellen Benutzerpräferenz für ein medizinisches Gerät (140), mit den Schritten:

    Identifizieren eines speziellen Benutzers;
    Gewinnen wenigstens einer Vorgabeparametereinstellung in Verbindung mit Präferenzen für den identifizierten Benutzer;
    Anpassen der Einstellungen des medizinischen Gerätes (140), so dass sie den Benutzerpräferenzen entsprechen;
    Überwachen eines momentanen Zustandes der Parametereinstellungen für das medizinische Gerät (140);
    Erkennen, wenn die Parametereinstellungen für das medizinische Gerät (140) geändert werden;
    Ermitteln, ob die Parameteränderung ein Überschreiben einer Vorgabeparametereinstellung darstellt; und wenn eine Änderung ein Überschreiben darstellt, Aktualisieren der wenigstens einen Parametereinstellung gemäß der Änderung,
    **dadurch gekennzeichnet, dass**:

        die Ermittlung, ob eine Parameteränderung ein Überschreiben einer Vorgabeparametereinstellung darstellt, auf patientencharakteristischer Information basiert.

10. Verfahren nach Anspruch 9, wobei die Parametereinstellungen in mehrere Kategorien unterteilt sind, wovon wenigstens eine Kategorie Parameter in Verbindung mit Benutzerspezifischen Eigenheiten enthält und eine weitere Kategorie davon Parameter in Verbindung mit patientenabhängigen Eigenschaften enthält, und ein Computer (132) die Benutzerpräferenzeinstellung für jede Kategorie unterschiedlich neu berechnet.

11. Verfahren nach Anspruch 9, wobei Einstellparameter entweder gleich bleibend oder variabel sind, und ein Computer (132) die Benutzerpräferenzeinstellung für jeden Parametertyp unterschiedlich neu berechnet.

12. Verfahren nach Anspruch 11, wobei gleich bleibende Parameter unter Verwendung eines Alpha-Filters aktualisiert werden.

**Revendications**

1.  Système automatisé (100) pour commander des réglages de paramètre d'un dispositif médical (140) comprenant :

        un dispositif médical (140) comportant des réglages ajustables ;
        un module (110) de reconnaissance d'utilisateur pouvant fonctionner pour identifier un utilisateur ;
        une base (120) de données pour stocker au moins un réglage de paramètre associé à des préférences particulier à un utilisateur pour une configuration dudit dispositif médical (140) ;
        un dispositif (130) de réglage pour ajuster automatiquement ledit dispositif médical (140) conformément à des réglages préférentiels particuliers à un utilisateur en fonction dudit au moins un réglage de paramètre ; et
        un ordinateur (132) pouvant fonctionner pour détecter chaque fois que l'utilisateur change les réglages préférentiels de l'utilisateur et met à jour ledit au moins un réglage de paramètre en fonction d'une valeur d'un paramètre de changement, **caractérisé en ce que** :

> l'ordinateur peut fonctionner pour détecter les modifications de l'utilisateur en déterminant si une modification de paramètre constitue un changement en fonction d'informations caractéristiques sur le patient.

**2.** Système automatisé (100) selon la revendication 1, comprenant en outre un commutateur de changement (135) fonctionnant pour donner des instructions audit dispositif (130) de réglage pour déterminer s'il faut ajuster ledit au moins un réglage de paramètre lorsque l'utilisateur modifie un réglage de paramètre.

**3.** Système automatisé (100) selon la revendication 1, dans lequel ladite base (120) de données est située à distance du dispositif médical (140).

**4.** Système automatisé (100) selon la revendication 3, dans lequel ladite base (120) de données peut fonctionner pour communiquer avec le dispositif médical (140) par internet.

**5.** Système automatisé (100) selon la revendication 3, dans lequel ladite base (120) de données peut fonctionner pour stocker un seul réglage préférentiel d'utilisateur pour des réglages qui sont communs aux multiples dispositifs médicaux.

**6.** Système automatisé (100) selon la revendication 1, dans lequel lesdits réglages de paramètre sont divisés en de multiples catégories, dont au moins une catégorie contient des paramètres associés à des traits spécifique de l'utilisateur et dont une autre catégorie contient des paramètres associés à des caractéristiques dépendant du patient, et dans lequel ledit ordinateur (132) peut fonctionner pour recalculer ledit réglage préférentiel de l'utilisateur de façon différente pour chaque catégorie.

**7.** Système automatisé (100) selon la revendication 1, dans lequel des paramètres de réglage sont soit continus soit variables, et dans lequel ledit ordinateur (132) peut fonctionner pour recalculer ledit réglage préférentiel de l'utilisateur de façon différente pour chaque type de paramètre.

**8.** Système automatisé (100) selon la revendication 7, dans lequel des paramètres continus sont mis à jour en utilisant un filtre alpha.

**9.** Procédé pour commander automatiquement au moins un réglage de paramètre d'un dispositif médical (140) associé à une préférence d'un utilisateur individuelle pour un dispositif médical (140), comprenant :

> identifier un utilisateur particulier ;
> obtenir au moins un réglage de paramètre par défaut associé à des préférences de l'utilisateur identifié ;
> ajuster les réglages du dispositif médical (140) pour respecter les préférences de l'utilisateur ;
> surveiller un état actuel des réglages de paramètre pour le dispositif médical (140) ;
> détecter le moment où les réglages de paramètre sont modifiés pour ledit dispositif médical (140) ;
> déterminer si une modification de paramètre constitue un changement du réglage de paramètre par défaut ; et lorsqu'une modification constitue un changement, mettre à jour ledit au moins un réglage de paramètre conformément à la modification, **caractérisé en ce que**:

> > déterminer si une modification de paramètre constitue un changement d'un réglage de paramètre par défaut en fonction d'informations caractéristiques sur le patient.

**10.** Procédé selon la revendication 9, dans lequel lesdits réglages de paramètre sont divisés en de multiples catégories, dont au moins une catégorie contient des paramètres associés à des traits spécifiques à l'utilisateur et dont une autre catégorie contient des paramètres associés à des caractéristiques dépendant du patient, et un ordinateur (132) recalcule ledit réglage préférentiel de l'utilisateur de façon différente pour chaque catégorie.

**11.** Procédé selon la revendication 9, dans lequel des paramètres de réglage sont soit continus soit variables, et dans lequel un ordinateur (132) recalcule ledit réglage préférentiel de l'utilisateur de façon différente pour chaque type de paramètre.

**12.** Procédé selon la revendication 11, dans lequel des paramètres continus sont mis à jour en utilisant un filtre alpha.

# FIG. 1

EP 1 199 984 B1

EP 1 199 984 B1

# FIG. 2

200 — Recognize User

202 — Obtain User's stored default parameters and adjust the medical device based on the stored defaults

204 — Monitor Use

206 — Is the User changing the default parameters?

No

Yes

210 — Update Defaults

208 — Is the change an override ?

Yes

No

212 — Continue

11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63310078 A
- JP 02004350 A
- JP 10234730 A

**Non-patent literature cited in the description**

- *PATENT ABSTRACTS OF JAPAN,* 31 December 1998, vol. 1998, 14